# EUROPÄISCHE PATENTSCHRIFT

(11) **EP 0 529 498 B1**
(45) Veröffentlichungstag und Bekanntmachung des Hinweises auf die Patenterteilung: **22.11.1995**
(21) Anmeldenummer: 92114144.6
(22) Anmeldetag: 19.08.1992
(51) Int. Cl.: C07C 227/16, C07C 229/58, C07C 229/62

(54) **Verfahren zur Herstellung von 2,5-Di-(phenylamino)-terephthalsäure und ihrer Dialkylester in hoher Reinheit**
Process for the preparation of 2,5-di-(phenylamino)-terephthalic acid and its dialkylesters in high purity
Procédé pour la préparation de l'acide 2,5-di-(phénylamino)-téréphthalique et son esters en haute pureté

(30) Priorität: 22.08.1991 DE 4127736
(43) Veröffentlichungstag der Anmeldung: 03.03.1993
(73) Patentinhaber: HOECHST AKTIENGESELLSCHAFT, 65926 Frankfurt am Main (DE)
(72) Erfinder: Ritter, Eberhard, Dr., W-6082 Mörfelden-Walldorf (DE); Schäfer, Hans, W-6232 Bad Soden am Taunus (DE); Vollheim, Thomas, Dr., W-6450 Hanau 1 (DE); Schottler, Martin, Dr., W-6200 Wiesbaden (DE)

(56) Entgegenhaltungen:
- EP-A- 0 363 756

## Beschreibung

2,5-Di-(phenylamino)-terephtalsäuren sind wichtige Zwischenprodukte für die Herstellung von Chinacridonpigmenten.

Die vorliegende Erfindung betrifft ein technologisch vorteilhaftes Verfahren zur Herstellung von 2,5-Di-(phenylamino)-terephthalsäure und ihrer Dialkylester der allgemeinen Formel (I) 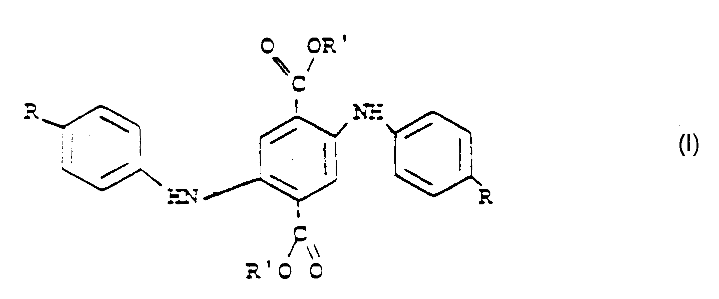
in welcher R ein Wasserstoffatom oder eine Methylgruppe und R' ein Wasserstoffatom oder eine Methyl- oder Ethylgruppe bedeuten.

Es ist bekannt, 2,5-Di-(phenylamino)-terephthalsäure und deren Dialkylester nach einem Mehrstufenverfahren herzustellen, indem man nach Art einer Dieckmann-bzw. doppelten Claisen-Kondensation Bernsteinsäure-dialkylester zum 2,5-Dihydroxy-cyclohexadien-dicarbonsaure-(1,4)-dialkylester cyclisiert (Fortschr. chem. Forschung, Bd. 1 (1950) 685-724), anschließend diesen in den 2,5-Di-phenylamino-dihydro(3,6)-terephthalsäure-dialkylester durch eine Kondensationsreaktion mit einem primären Phenylamin (beispielsweise Anilin oder Toluidin) in Xylol oder Ethylbenzol oder in Gemischen daraus in Gegenwart einer aliphatischen Säure (beispielsweise Essigsäure) überführt, diesen dehydriert (oxidiert) zum 2,5-Di-(phenylamino)-terephthalsäure-dialkylester, anschließend diesen Ester alkalisch verseift (z. B. in alkoholischer Natronlauge) und durch Behandeln des angefallenen 2,5-di-(phenylamino)-terephthalsauren Dinatriumsalzes mit Säure die 2,5-Di-(phenylamino)-terephthalsäure freisetzt.

Bei der Beschreibung der Herstellung der Di-(phenylamino)-terephthalsäure nach dem vorstehend beschriebenen Weg aus Bernsteinsäureester werden in der Literatur (JP 49-108 036; US-PS 35 55 087 und US-PS 4 981 997 (EP 0 363 756)), eine Reihe von Verfahrensparametern beschrieben, wie beispielsweise Lösemittel;
die Zwischenisolierung einzelner oder aller Synthesestufenprodukte (wie (1) Succinylo-bernsteinsäure-dialkylester;
(2) 2,5-Di-(phenylamino)-dihydro(3,6)-terephthalsäure-dialkylester;
(3) 2,5-Di-(phenylamino)-terephthalsäure-dialkylester;
(4) 2,5-Di-(phenylamino)-terephthalsäure); die Art der angewandten Katalysatoren, gegebenenfalls mit Zusatzstoffen für die vorstehend genannten Zwischenstufen (1), (2) und (3); zeitliche Abfolge von Oxidation und Verseifung (Verseifung gleichzeitig mit Oxidation oder anschließend); Dehydrierungs-(Oxidations)mittel (wie beispielsweise Nitrobenzol und dessen Derivate, Chinone, Sauerstoff, Jod);
Aufarbeitung der eingesetzten Hilfsstoffe (wie beispielsweise Lösemittel, Phenylamin (Anilin, p-Toluidin), Katalysatoren, Zusatzstoffe).

Gegenstand der Erfindung ist nun ein Verfahren zur Herstellung von 2,5-Di-(phenylamino)-terephtalsäure-dialkylestern der allgemeinen Formel (I) (siehe Patentanspruch 1), in welcher R ein Wasserstoffatom oder eine Methylgruppe und R' eine Methyl- oder Ethylgruppe bedeuten, durch Dehydrierung (Oxidation) des entsprechenden 2,5-Di-(phenylamino)-dihydro(3,6)-terephtalsäuredialkylester mit Sauerstoff, das dadurch gekennzeichnet ist, daß man eine Lösung oder Suspension des 2,5-Di-(phenylamino)-dihydro(3,6)-terephtalsäuredialkylesters in aromatischen Kohlenwasserstoffen in einem Rührkessel mit Sauerstoff überlagert, dieses Reaktionsgemisch im Kreislauf über eine Sprühvorrichtung fördert, so daß das versprühte Reaktionsgemisch über dem im Rührkessel vorliegenden Reaktionsgemisch verteilt wird, wobei das versprühte Reaktionsgemisch mit dem Kreisgas gemischt wird.

Das Ausgangsprodukt 2,5-Di-(phenylamino)-dihydro(3,6)-terephthalsäuredialkylester kann nach üblichen Verfahren hergestellt werden. Auch die Weiterverarbeitung des Produkts zum Dinatrium-Salz und gegebenenfalls zur entsprechenden Säure kann vorteilhaft entsprechend dieser Anmeldung erfolgen.

Ein weiterer Gegenstand der vorliegenden Erfindung ist deshalb ein Verfahren zur Herstellung von 2,5-Di-(phenylamino)-terephthalsäure und ihrer Dialkylester der allgemeinen Formel (I) (s. Patentanspruch 2), in welcher R ein Wasserstoffatom oder eine Methylgruppe und R' ein Wasserstoffatom oder eine Methyl- oder Ethylgruppe bedeuten, durch (1) Umsetzung von Bernsteinsäure-dialkyl(C₁-C₂)-ester nach Art einer Dieckmann-Kondensation mit Natrium-alkoholat in Xylol zum Dinatrium-Salz des 2,5-Dihydroxy-cyclohexadien-dicarbonsäure-(1,4)-dialkyl(C₁-C₂)esters, (2) Umsetzung des so erhaltenen Kondensationsprodukts nach Zersetzen des Dinatrium-Salzes mit Säure mit einem Phenylamin der allgemeinen Formel (II) (s. Patentanspruch 2), in welcher R die vorstehend genannte Bedeutung hat, in Gegenwart einer organischen Säure in aromatischen Kohlenwasserstoffen zum 2,5 Di-(phenylamino)-dihydro(3,6)-terephthalsäure-dialkyl(C₁-C₂)-ester, (3) Dehydrierung (Oxidation) des so erhaltenen Cyclohexadien-1,4-derivats mit Sauerstoff zum entsprechenden 2,5-Di-(phenylamino)-terephthalsäure-dialkyl(C₁-C₂)-ester, (4) Verseifung des so erhaltenen Dialkylesters in methanolischer Natronlauge zum entsprechenden 2,5-di-(phenylamino)-terephthalsauren Dinatrium-Salz und (5) Freisetzung der 2,5-Di-(phenylamino)-terephthalsäure aus dem genannten Dinatriumsalz mit Säure, das dadurch gekennzeichnet ist, daß man die Oxidation in Stufe (3) so durchführt, daß man das als Lösung oder Suspension vorliegende Reaktionsgemisch der Stufe (2) in einem Rührkessel mit Sauerstoff überlagert, dieses Reaktionsgemisch im Kreislauf über eine Sprühvorrichtung fördert, so daß das versprühte Reaktionsgemisch über dem im Rührkessel vorliegenden Reaktionsgemisch verteilt wird, wobei das versprühte Reaktionsgemisch mit dem Kreisgas gemischt wird.

Der Sauerstoff kann allein oder im Gemisch mit inerten Gasen, z.B. in Form von Luft verwendet werden. Als aromatische Lösungsmittel eignen sich insbesondere die verschiedenen Xylol-Isomere und ganz allgemein solche in denen der Sauerstoff ähnlich gut löslich ist wie in Xylol. Diese Lösungsmittel sollten im allgemeinen mit Wasser ein Azeotrop bilden, wobei das azeotrope Gemisch bei unter etwa 120°C sieden sollte. Geeignet sind somit beispielsweise Toluol, Chlorbenzol, Ethylbenzol, Diisopropylnaphthalin und Trialkylbenzole. Besonders bevorzugt ist Xylol allein oder technische Xylolgemische.

Für die Umsetzung mit dem Phenylamin der Formel (II) in Stufe (2) kann beispielsweise Propionsäure oder Hexafluorpropansulfonsäure als Säurekatalysator verwendet werden.

Bei dem Kreisgas handelt es sich im allgemeinen um ein Gemisch aus einerseits dem sich über dem vorgelegten Reaktionsgemisch im dynamischen Gleichgewicht einstellenden Gasgemisch mit dem andererseits gegebenenfalls mit Inertgasen gemischten Sauerstoff, mit dem das Reaktionsgemisch überlagert wird, und der nach Bedarf nachdosiert wird.

Die Zeit, in der das Reaktionsgemisch einmal umgepumpt wird beträgt im allgemeinen 0,5 bis 10 min, insbesondere 1 bis 6 min.

Das erfindungsgemäße Verfahren wird zweckmäßig bei einer Temperatur des Reaktionsgemisches von 80 bis 120°C, vorzugsweise von 90 bis 110°C, insbesondere von 95 bis 100°C, durchgeführt. Bei höheren Drucken als Atmosphärendruck können auch höhere Temperaturen angewendet werden.

Bei der Sprühvorrichtung handelt es sich im allgemeinen um eine Vorrichtung, die Öffnungen in Form von beispielsweise Löchern, Schlitzen oder Kanälen enthält, durch die die zu verteilende Flüssigkeit gedrückt wird. Dabei kann es sich insbesondere um Lochbleche oder um eine oder mehrere Düsen enthaltende Vorrichtung, wie Vollkegel-, Ring- oder Ejektordüsen enthaltende Vorrichtung, handeln. Ejektordüsen sind selbstansaugende Düsen, deren Vorteil darin besteht, daß die gewünschte Strömungsgeschwindigkeit des Kreisgases durch den Saugeffekt der Düse erzeugt wird, so daß es ohne zusätzlichen Gasverdichter gefördert werden kann. Bei anderen Düsen kann die jeweilige Strömungsgeschwindigkeit durch einen Verdichter, beispielsweise eine Pumpe, der das Kreisgas fördert, bewirkt werden.

Das Reaktorvolumen und die Menge des vorgelegten Reaktionsgemisches, die Geschwindigkeit, mit der das Reaktionsgemisch umgepumpt wird und die Art, Anzahl und Dimension der Strahldüsen der Sprühvorrichtung werden zweckmäßig so gewählt, daß die spezifische Phasengrenzfläche 200 bis 7000 m²/m³, vorzugsweise 300 bis 6000 m²/m³, besonders bevorzugt 400 bis 5000 m²/m³, bezogen auf das Reaktorvolumen, beträgt. Die Phasengrenzfläche läßt sich insbesondere durch die Größe und Anzahldichte der versprühten Tropfen beeinflussen.

Ein Vorteil des erfindungsgemäßen Verfahrens ist die relativ kurze Reaktionszeit verbunden mit günstigen Raum-Zeit-Ausbeuten und apparativer Einfachheit. Dies beruht unter anderem darauf, daß durch das Versprühen des Reaktionsgemisches im Kreisgas ein höherer Gehalt an gelöstem Sauerstoff in der Reaktionslösung gewährleistet ist, so daß der Einsatz zusätzlicher Katalysatoren entfallen kann, was einen besonderen Vorteil darstellt. Es können aber auch Katalysatoren aus V₄A-Stahl und/oder einem Übergangsmetall des Periodischen Systems der Elemente und/oder aus einem seltenen Erdmetall mit variablen Oxidationsstufen oder dessen Verbindungen eingesetzt werden.

Die Beschleunigung der Reaktion erfolgt außer durch die Schaffung einer großen Phasengrenzfläche flüssig-gasförmig auch durch die Verringerung der Diffusions-Grenzschichtdicke an den Feststoffteilchen der suspendierten Ausgangsverbindung, durch die beim Sprühvorgang auftretenden Scherkräfte. So werden die Stofftransportwiderstände verringert und die Stoffaustauschfläche vergrößert.

Da die Reaktionszeit auch vom Sauerstoffpartialdruck des Kreisgases abhängig ist, läßt sie sich auf diese Weise steuern, so daß optimale Taktzeiten eingehalten werden können. Sie läßt sich in weiten Grenzen einstellen. Im allgemeinen werden Reaktionszeiten von 3 bis 16 h eingehalten.

Der Sauerstoffpartialdruck kann auch über eine Änderung des Gesamtdrucks der Gasphase im Reaktor gesteuert werden. Der Gesamtdruck liegt dabei im allgemeinen zwischen 1 und 10 bar, insbesondere bei Atmosphärendurck.

Die erhöhte Reaktionsgeschwindigkeit ist insbesondere deshalb von Vorteil, weil dadurch optimale Taktzeiten für die jeweilige Weiterverarbeitung beim Übergang von Stufe (2) nach (3) und weiter nach Stufe (4) erreicht werden.

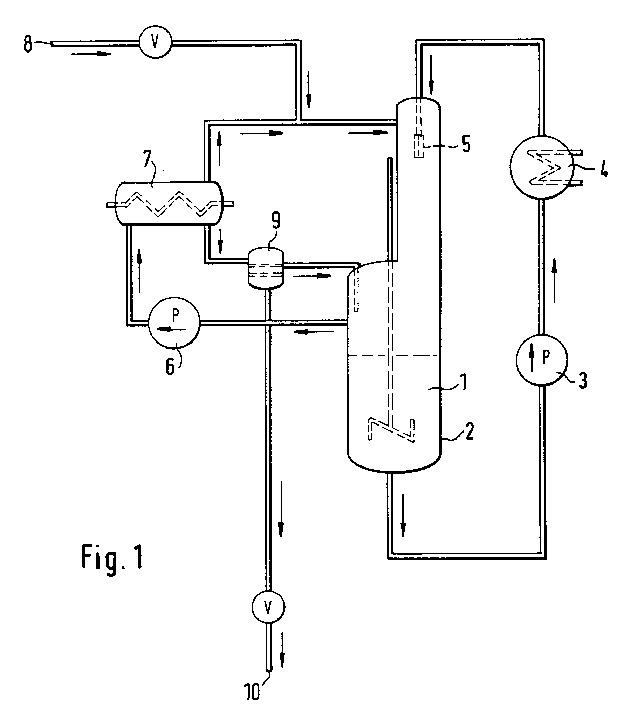

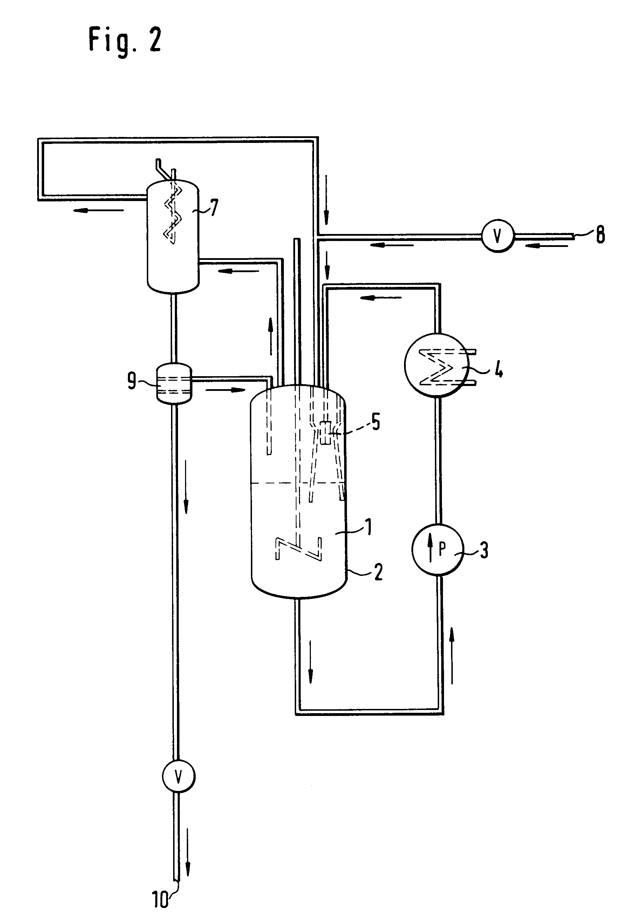

Das erfindungsgemäße Verfahren wird anhand der Figuren 1 und 2 beispielhaft erläutert:

### Figur 1

Die Reaktionsmischung 1 wird in einem Rührgefäß 2 vorgelegt und durch den Bodenablaß entnommen und von der Pumpe 3 durch den Wärmetauscher 4 zur Düse 5 gefördert, und dort versprüht. Die Sprühdüse ist in einem auf den Reaktor aufgesetzten, turmartigen Aufbau montiert. Die versprühte Flüssigkeit läuft zurück in das Rührgefäß 2. Das Kreisgas wird aus dem Gasraum über der Reaktionsmischung durch eine Pumpe 6 entnommen und über den Kondensor 7 zurück in den Reaktor 2 gefördert, wobei zur Ergänzung des verbrauchten Sauerstoffs eine Nachdosierung 8 von Sauerstoff und gegebenenfalls Inertgas angebracht ist. Das im Kondensor 7 kondensierte Dampfgemisch wird in einen Phasenscheider 9 geleitet, von wo aus die leichte Phase in den Reaktor 2 zurückfließt, und die schwere (Wasser-) Phase aus der Apparatur ausgeschleust wird.

### Figur 2

Der Kreislauf für das Reaktionsgemisch ist gleich aufgebaut wie in Figur 1. Die Düse 5 befindet sich jedoch innerhalb des als Vorlage dienenden Kessels 2 und ist selbstansaugend, so daß im Gaskreislauf die Förderpumpe für das Gas entfällt.

Ansonsten ist auch der Gaskreislauf gleich aufgebaut, und enthält eine Nachdosier-Einrichtung 8 für Sauerstoff und eine Ausschleusung 10 des in der Reaktion gebildeten Wassers.

### Beispiele

1. In einem als Vorlage dienenden Rührkessel mit 5 l Volumen wurde eine Suspension des 2,5-Diphenylamino-dihydro(3,6)-terephthalsäure-dimethylesters in Xylol vorgelegt. Sie enthielt 736 g des Dihydroesters, 360 g Propionsäure, 72 g Anilin und 1850 g Xylol. Propionsäure und Anilin stammten aus den vorhergehenden Reaktionsschritten. Die Suspension wurde über einen Wärmetauscher gepumpt und über eine Vollkegeldüse mit einem Sprühwinkel von 15° in einem zylindrischen Rohr versprüht. Die versprühte Flüssigkeit wurde wieder in die Vorlage zurückgeleitet. Die Geschwindigkeit, mit der die Flüssigkeit umgepumpt wurde, betrug 180 l/h, der Durchmesser der Düsenbohrung betrug 1,6 mm. Die Phasengrenzfläche, bezogen auf das Reaktorvolumen, betrug 400 m²/m³. Nach Erreichen einer Temperatur von 97°C wurde das Kreisgas eingeschaltet. Der Gasstrom betrug 100 l/h. Zur Oxidation wurde Luft verwendet, die zur Entfernung des bei der Reaktion entstehenden Wassers über einen Kühler geführt wurde. Das Kondensat (Wasser/Xylol) wurde in einem Phasenscheider getrennt, das Wasser ausgeschleust und das Xylol in den Reaktor zurückgeführt. Die Reaktion war nach 4,5 h beendet, die Ausbeute an Produkt betrug 99 % (HPLC-Analyse als Flächen-Prozente).
2. Es wurde wie in Beispiel 1 verfahren, jedoch wurde von einer Suspension von 2,5-Di-(p-toluidino)-dihydro(3,6)-terephthalsäuredimethylester ausgegangen. Die Suspension enthielt 812 g des Dihydroesters, 360 g Propionsäure, 43 g p-Toluidin und 1850 g Xylol. Die Reaktion war nach 6,5 h beendet, die Ausbeute an Produkt betrug 99 %.
3. Beispiel 1 wurde wiederholt, wobei jedoch statt Luft eine Stickstoff/Sauerstoff-Mischung mit 40 Vol% Sauerstoff benutzt wurde. Die Reaktion war nach 3,5 h beendet, die Ausbeute an Produkt betrug 97%.
4. In einem als Vorlage dienenden Rührkessel mit 370 l Volumen wurde eine Suspension des 2,5-Di-(p-toluidino)-dihydro(3,6)-terephthalsäure-dimethylesters in Xylol vorgelegt. Sie enthielt 65 kg des Dihydroesters, 26 kg Propionsäure, 6,5 kg p-Toluidin und 250 kg Xylol. Propionsäure und p-Toluidin stammten aus den vorhergehenden Reaktionsschritten.
   Die Suspension wurde über einen Wärmetauscher gepumpt und über eine Vollkegeldüse mit einem Sprühwinkel von 15° in einem zylindrischen Rohr versprüht. Die versprühte Flüssigkeit wurde wieder in die Vorlage zurückgeleitet. Die Geschwindigkeit, mit der die Flüssigkeit umgepumpt wurde, betrug 8000 l/h, der Durchmesser der Düsenbohrung 12 mm. Die Phasengrenzfläche, bezogen auf das Reaktorvolumen, betrug 2800 m²/m³.
   Nach Erreichen einer Temperatur von 97°C wurde der Gasstrom eingeschaltet. Der Gasstrom betrug 15000 l/h. Zur Oxidation wurde ein Stickstoff/Sauerstoff-Gemisch mit 8 Vol% Sauerstoff verwendet. Die Reaktion war nach 13 h beendet, die Ausbeute an Produkt betrug 99 % (HPLC-Analyse als Flächen-Prozente).

## Patentansprüche

1. Verfahren zur Herstellung von 2,5-Di-(phenylamino)-terephthalsäure-dialkylestern der Formel (I) 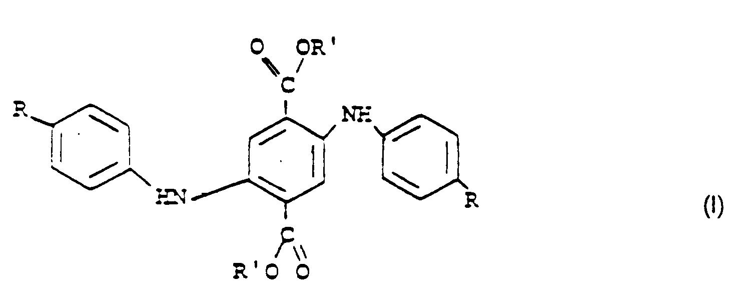 in welcher R ein Wasserstoffatom oder eine Methylgruppe und R' eine Methyl-oder Ethylgruppe bedeuten, durch Dehydrierung (Oxidation) des entsprechenden 2,5-Di-(phenylamino)-dihydro(3,6)-terephthalsäuredialkylesters mit Sauerstoff, dadurch gekennzeichnet, daß man eine Lösung oder Suspension des 2,5-Di-(phenylamino)-dihydro(3,6)-terephthalsäuredialkylesters in aromatischen Kohlenwasserstoffen in einem Rührkessel mit Sauerstoff überlagert, dieses Reaktionsgemisch im Kreislauf über eine Sprühvorrichtung fördert, so daß das versprühte Reaktionsgemisch über dem im Rührkessel vorliegenden Reaktionsgemisch verteilt wird, wobei das versprühte Reaktionsgemisch mit dem Kreisgas gemischt wird.

2. Verfahren zur Herstellung von 2,5-Di-(phenylamino)-terephthalsäure und ihrer Dialkylester der allgemeinen Formel (I), 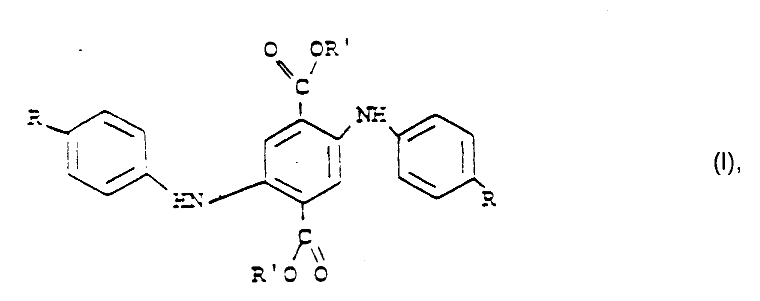 in welcher R ein Wasserstoffatom oder eine Methylgruppe und R' ein Wasserstoffatom oder eine Methyl- oder Ethylgruppe bedeuten, durch (1) Umsetzung von Bernsteinsäure-dialkyl(C₁-C₂)-ester nach Art einer Dieckmann-Kondensation mit Natrium-alkoholat in Xylol zum Dinatrium-Salz des 2,5-Dihydroxycyclohexadien-dicarbonsäure-(1,4)-dialkyl(C₁-C₂)esters, (2) Umsetzung des so erhaltenen Kondensationsprodukts nach Zersetzen des Dinatrium-Salzes mit Säure mit einem Phenylamin der allgemeinen Formel (II) 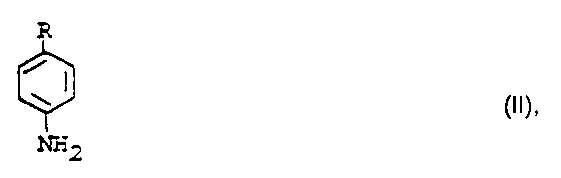 in welcher R die vorstehend genannte Bedeutung hat, in Gegenwart einer organischen Säure in aromatischen Kohlenwasserstoffen zum 2,5-Di-(phenylamino)-dihydro(3,6)-terephthalsäure-dialkyl(C₁-C₂)-ester, (3) Dehydrierung (Oxidation) des so erhaltenen Cyclohexadien-1,4-derivats mit Sauerstoff zum entsprechenden 2,5-Di-(phenylamino)-terephthalsäure-dialkyl(C₁-C₂)-ester, (4) Verseifung des so erhaltenen Dialkylesters in methanolischer Natronlauge zum entsprechenden 2,5-di-(phenylamino)-terephthalsauren Dinatrium-Salz und (5) Freisetzung der 2,5-Di-(phenylamino)-terephthalsäure aus dem genannten Dinatriumsalz mit Säure, dadurch gekennzeichnet, daß man die Oxidation in Stufe (3) so durchführt, daß man das als Lösung oder Suspension vorliegende Reaktionsgemisch der Stufe (2) in einem Rührkessel mit Sauerstoff überlagert, dieses Reaktionsgemisch im Kreislauf über eine Sprühvorrichtung fördert, so daß das versprühte Reaktionsgemisch über dem im Rührkessel vorliegenden Reaktionsgemisch verteilt wird, wobei das versprühte Reaktionsgemisch mit dem Kreisgas gemischt wird.

3. Verfahren nach Anspruch 1 oder 2, dadurch gekennzeichnet, daß der Sauerstoff in Form von Luft eingesetzt wird.

4. Verfahren nach einem oder mehreren der Ansprüche 1 bis 3, dadurch gekennzeichnet, daß die Zeit, in der das Reaktionsgemisch einmal umgepumpt wird, 0,5 bis 10 min, vorzugsweise 1 bis 6 min, beträgt.

5. Verfahren nach einem oder mehreren der Ansprüche 1 bis 4, dadurch gekennzeichnet, daß die Sprühvorrichtung eine oder mehrere Düsen enthält.

6. Verfahren nach Anspruch 5, dadurch gekennzeichnet, daß die Düse eine Vollkegel-, Ring- oder Ejektordüse ist.

7. Verfahren nach Anspruch 5, dadurch gekennzeichnet, daß die Düse selbstansaugend ist.

8. Verfahren nach einem oder mehreren der Ansprüche 1 bis 7, dadurch gekennzeichnet, daß die Reaktion bei Temperaturen von 80 bis 120°C, vorzugsweise von 90 bis 110°C, besonders bevorzugt von 95 bis 100°C, durchgeführt wird.

9. Verfahren nach einem oder mehreren der Ansprüche 1 bis 8, dadurch gekennzeichnet, daß die spezifische Phasengrenzfläche 200 bis 7000 m²/m³, vorzugsweise 300 bis 6000 m²/m³, besonders bevorzugt 400 bis 5000 m²/m³, bezogen auf das Reaktorvolumen, beträgt.

10. Verfahren nach einem oder mehreren der Ansprüche 1 bis 9, dadurch gekennzeichnet, daß der Gesamtdruck der Gasphase im Reaktor zwischen 1 und 10 bar, bevorzugt bei Atmosphärendruck, liegt.

## Claims

1. A process for the preparation of a dialkyl 2,5-di(phenylamino)terephthalate of the formula (I) 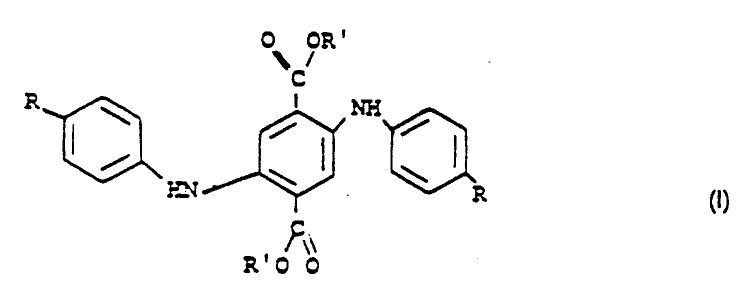 in which R is a hydrogen atom or a methyl group and R' is a methyl group or ethyl group, by dehydrogenation (oxidation) of the corresponding dialkyl 2,5-di(phenylamino)-3,6-dihydroterephthalate with oxygen, which comprises blanketing with oxygen a solution or suspension of the dialkyl 2,5-di(phenylamino)-3,6-dihydroterephthalate in aromatic hydrocarbons in a stirred vessel, circulating this reaction mixture via a spraying device, so that the sprayed reaction mixture is distributed over the reaction mixture present in the stirred vessel, the sprayed reaction mixture being mixed with the circulating gas.

2. A process for the preparation of 2,5-di(phenylamino)terephthalic acid and a dialkyl ester thereof of the formula (I), 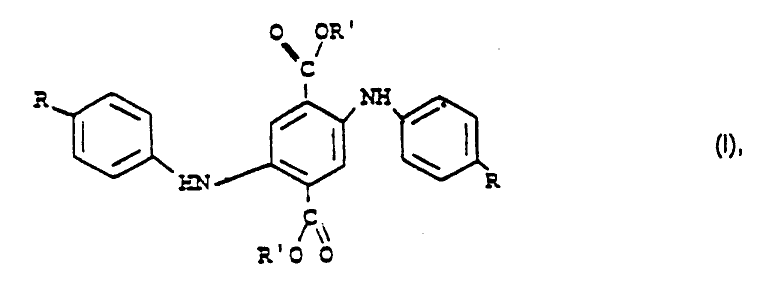 in which R is a hydrogen atom or a methyl group and R' is a hydrogen atom or a methyl group or ethyl group, by (1) reacting di(C₁-C₂)alkyl succinate with sodium alcoholate in xylene in the manner of a Dieckmann condensation to give the disodium salt of the di(C₁-C₂)alkyl 2,5-dihydroxycyclohexadiene-1,4-dicarboxylate, (2) reacting the condensation product thus obtained, after decomposition of the disodium salt by means of acid, with a phenylamine of the formula (II) 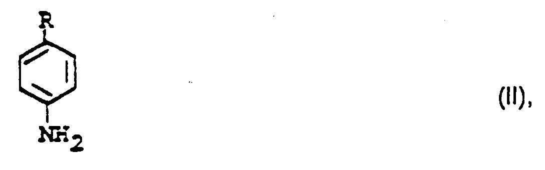 in which R has the abovementioned meaning, in the presence of an organic acid in aromatic hydrocarbons to give the di(C₁-C₂)alkyl 2,5-di(phenylamino)-3,6-dihydroterephthalate, (3) dehydrogenating (oxidizing) the cyclo-1,4-hexadiene derivative thus obtained with oxygen to give the corresponding di(C₁-C₂)alkyl 2,5-di(phenylamino)terephthalate, (4) hydrolyzing the dialkyl ester thus obtained in methanolic sodium hydroxide solution to give the corresponding disodium salt of 2,5-di(phenylamino)terephthalic acid and (5) liberating the 2,5-di(phenylamino)terephthalic acid from the said disodium salt using acid, which comprises carrying out the oxidation in stage (3) in such a manner that the reaction mixture of stage (2) present as solution or suspension is blanketed with oxygen in a stirred vessel, this reaction mixture is circulated via a spraying device, so that the sprayed reaction mixture is distributed over the reaction mixture present in the stirred vessel, the sprayed reaction mixture being mixed with the circulating gas.

3. The process as claimed in claim 1 or 2, wherein the oxygen is used in the form of air.

4. The process as claimed in one or more of claims 1 to 3, wherein the time in which the reaction mixture is circulated once is 0.5 to 10 min, preferably 1 to 6 min.

5. The process as claimed in one or more of claims 1 to 4, wherein the spraying device contains one or more nozzles.

6. The process as claimed in claim 5, wherein the nozzle is a solid cone nozzle, ring nozzle or ejector nozzle.

7. The process as claimed in claim 5, wherein the nozzle is self-priming.

8. The process as claimed in one or more of claims 1 to 7, wherein the reaction is carried out at temperatures of from 80 to 120°C, preferably from 90 to 110°C, particularly preferably from 95 to 100°C.

9. The process as claimed in one or more of claims 1 to 8, wherein the specific interfacial area is 200 to 7000 m²/m³, preferably 300 to 6000 m²/m³, particularly preferably 400 to 5000 m²/m³, relative to the reactor volume.

10. The process as claimed in one or more of claims 1 to 9, wherein the total pressure of the gas phase in the reactor is between 1 and 10 bar, preferably at atmospheric pressure.

## Revendications

1. Procédé de préparation d'esters dialkyliques de l'acide 2,5-di(phénylamino)téréphtalique de formule générale (I) 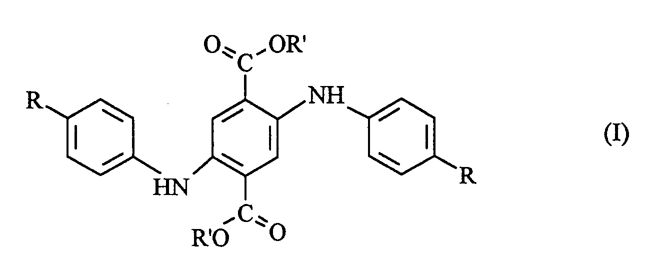 dans laquelle R représente un atome d'hydrogène ou un groupe méthyle et R' représente un groupe méthyle ou éthyle, par déshydrogénation (oxydation) de l'ester dialkylique de l'acide 2,5-di(phénylamino)-3,6-dihydrotéréphtalique correspondant avec de l'oxygène, caractérisé en ce que l'on recouvre d'oxygène une solution ou une suspension de l'ester dialkylique de l'acide 2,5-di(phénylamino)-3,6-dihydrotéréphtalique dans des solvants aromatiques dans un réacteur à agitation, on fait passer en circuit fermé ce mélange réactionnel par un dispositif de pulvérisation de façon que le mélange réactionnel pulvérisé se divise au-dessus du mélange réactionnel présent dans le réacteur à agitation, grâce à quoi le mélange réactionnel pulvérisé se mélange avec le gaz de circulation.

2. Procédé de préparation de l'acide 2,5-di(phénylamino)téréphtalique et de ses esters dialkyliques de formule générale (I) 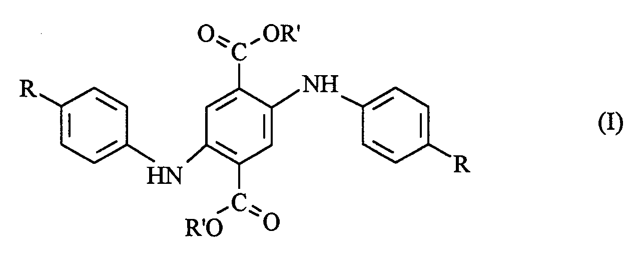 dans laquelle R représente un atome d'hydrogène ou un groupe méthyle et R' est un atome d'hydrogène ou un groupe méthyle ou éthyle, selon lequel (1) on fait réagir un ester de di(alkyle en C₁-C₂) de l'acide succinique selon une condensation de Dieckmann avec un alcoolate de sodium dans du xylène pour former le sel disodique de l'ester de di(alkyle en C₁-C₂) de l'acide 2,5-dihydroxycyclohexadiène-1,4-dicarboxylique, (2) on fait réagir le produit de condensation ainsi obtenu, après décomposition du sel disodique avec un acide, avec une phénylamine de formule générale (II) 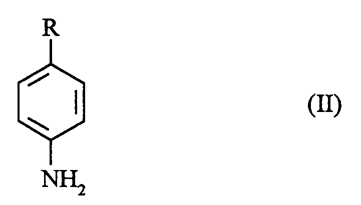 dans laquelle R a la signification donnée précédemment, en présence d'un acide organique dans des hydrocarbures aromatiques, pour donner l'ester de di(alkyle en C₁-C₂) de l'acide 2,5-di(phénylamino)-3,6-dihydrotéréphtalique, (3) on procède à une déshydrogénation (oxydation) du dérivé de 1,4-cyclohexadiène ainsi obtenu avec de l'oxygène pour former l'ester de di(alkyle en C₁-C₂) de l'acide 2,5-di(phénylamino)téréphtalique correspondant, (4) on saponifie l'ester dialkylique ainsi obtenu dans une solution méthanolique de soude pour former le sel disodique de l'acide 2,5-di(phénylamino)téréphtalique correspondant et (5) on libère l'acide 2,5-di(phénylamino)téréphtalique à partir dudit sel disodique avec un acide, caractérisé en ce que l'on effectue l'oxydation dans l'étape (3) en recouvrant d'oxygène le mélange réactionnel de l'étape (2) se trouvant sous forme d'une solution ou d'une suspension dans un réacteur à agitation et en faisant passer en circuit fermé ce mélange réactionnel par un dispositif de pulvérisation de façon que le mélange réactionnel pulvérisé se divise au-dessus du mélange réactionnel présent dans le réacteur à agitation, grâce à quoi le mélange réactionnel pulvérisé se mélange avec le gaz de circulation.

3. Procédé selon la revendication 1 ou 2, caractérisé en ce que l'on introduit l'oxygène sous forme d'air.

4. Procédé selon l'une ou plusieurs des revendications 1 à 3, caractérisé en ce que le temps pendant lequel on fait circuler une fois par pompage le mélange réactionnel est compris entre 0,5 et 10 minutes, de préférence entre 1 et 6 minutes.

5. Procédé selon l'une ou plusieurs des revendications 1 à 4, caractérisé en ce que le dispositif de pulvérisation contient une ou plusieurs buses.

6. Procédé selon la revendication 5, caractérisé en ce que la buse est une buse conique, annulaire ou à éjecteur.

7. Procédé selon la revendication 5, caractérisé en ce que la buse est à auto-amorçage.

8. Procédé selon l'une ou plusieurs des revendications 1 à 7, caractérisé en ce que la réaction est effectuée à des températures comprises entre 80 et 120°C, de préférence entre 90 et 110°C, de façon particulièrement préférée entre 95 et 100°C.

9. Procédé selon l'une ou plusieurs des revendications 1 à 8, caractérisé en ce que la surface spécifique de séparation des phases est comprise entre 200 et 7000 m²/m³, de préférence entre 300 et 6000 m²/m³, de façon particulièrement préférée entre 400 et 5000 m²/m³, par rapport au volume du réacteur.

10. Procédé selon l'une ou plusieurs des revendications 1 à 9, caractérisé en ce que la pression totale de la phase gazeuse dans le réacteur est comprise entre 1 et 10 bars, et est de préférence la pression atmosphérique.
